(19) Europäisches Patentamt
European Patent Office
Office européen des brevets

(11) Veröffentlichungsnummer: **0 043 448**
**B1**

(12) # EUROPÄISCHE PATENTSCHRIFT

(45) Veröffentlichungstag der Patentschrift: **18.01.84**

(21) Anmeldenummer: **81104306.6**

(22) Anmeldetag: **04.06.81**

(51) Int. Cl.³: **C 07 D307/12**, C 09 J 3/14, C 09 K 3/10

(54) **Bis-methacrylsäureester des 2,5-Dimethylol-tetrahydrofurans, Verfahren zu ihrer Herstellung und diese enthaltende Klebstoffe, beziehungsweise Dichtungsmittel.**

(30) Priorität: **12.06.80 DE 3021941**

(43) Veröffentlichungstag der Anmeldung: **13.01.82 Patentblatt 82/02**

(45) Bekanntmachung des Hinweises auf die Patenterteilung: **18.01.84 Patentblatt 84/03**

(84) Benannte Vertragsstaaten: **AT BE CH DE FR GB IT LI LU NL SE**

(56) Entgegenhaltungen: **US-A- 3 634 373**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **Gruber, Werner, Dr.**
**Oppelner Weg 7**
**D-4000 Düsseldorf 12 (DE)**

**EP 0 043 448 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Bis-methacrylsäureester des 2,5-Dimethylol-tetrahydrofurans, Verfahren zu ihrer Herstellung und diese enthaltende Klebstoffe, beziehungsweise Dichtungsmittel

Die Erfindung bezieht sich auf neue Bis-methacrylsäureester, die sich vom 2,5-Dimethylol-tetrahydrofuran ableiten. Sie betrifft weiterhin die Herstellung derartiger Verbindungen sowie diese enthaltende, Klebstoffe bzw. Dichtungsmittel.

Methacrylsäureester und zwar sowohl Monoester als auch Diester sind seit langem bekannt und können unter anderem als Basis für bei Sauerstoffausschluß spontan erhärtende Klebstoffe verwendet werden. So sind Bis-methacrylsäureester von propoxyliertem Diphenylolpropan sowie speziellen Derivaten von Tricyclodecan bereits für diesen Anwendungszweck eingesetzt worden.

Anaerob härtende Klebstoffe auf Basis dieser Bis-methacrylate lassen jedoch anwendungstechnisch noch Wünsche nach höheren Festigkeiten offen. Tetrahydrofurfurylmethacrylat ist ein Monomeres, mit dem anaerob härtende Klebstoffe mit hohen Festigkeiten bei Raumtemperatur herstellbar sind, jedoch ist aufgrund der relativ hohen Flüchtigkeit eine starke Geruchsbelästigung festzustellen. Außerdem ist die Wärmefestigkeit wegen der niedrigen Sealing-Temperatur des gebildeten Polymeren äußerst ungüngstig.

Aufgabe der Erfindung war es daher, neue, technisch wertvollere Bis-methacrylsäureester zu finden, die aufgrund ihrer Konstitution und ihrer Eigenschaften eine Verbesserung gegenüber dem bekannten Stand der Technik bringen.

Gegenstand der Erfindung sind Bis-methacrylsäureester der allgemeinen Formel

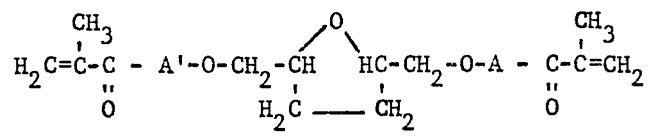

in der A beziehungsweise A' folgende Bedeutung haben

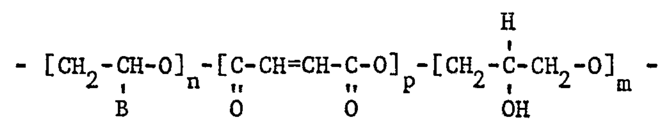

wobei

$B = CH_3$ oder H ist und n, p, m ganze Zahlen sind, die die Werte

$n = 0$ bis 5 und $m = 0$ oder 1 und $p = 0$ oder 1 annehmen können.

Für den einfachsten Fall, daß n, m und p 0 sind, liegt der Bis-methacrylsäureester von 2,5-Dimethylol-tetrahydrofuran vor ;

ist jedoch n 1 bei B = H, so erhält man :

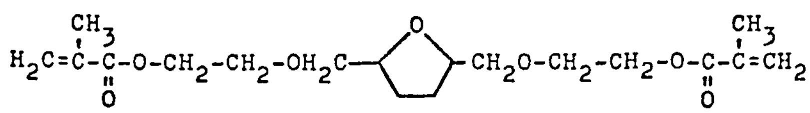

Bedeutet $n = 0$, $m = 1$ und $p = 1$, so erhält man folgende Verbindung :

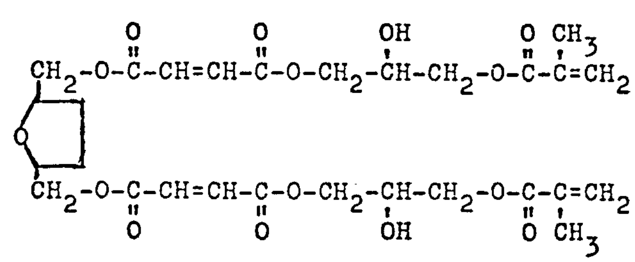

Wird auch $n = 1$, so resultiert :

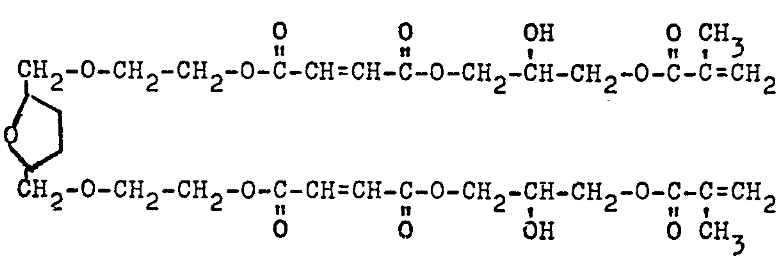

Die Herstellung der erfindungsgemäßen Bis-methacrylsäureester erfolgt mittels an sich bekannter Reaktion. So kann man 2,5-Dimethylol-tetrahydrofuran mit Methacrylsäure beziehungsweise dessen Chlorid beziehungsweise Methylester umsetzen. Weiter ist es möglich, 2,5-Dimethyl-ol-tetrahydrofuran mit Ethylenoxid und/oder Propylenoxid zu alkylieren und dann mit Methacrylsäure beziehungsweise dessen Chlorid beziehungsweise Methylester umzusetzen.

Ferner ist es möglich, die erfindungsgemäßen Bis-methacrylsäureester dadurch herzustellen, daß man 1 Mol 2,5-Dimethylol-tetrahydrofuran mit 1 bis 2 Mol Maleinsäureanhydrid in an sich bekannter Weise umsetzt und dann mit 2 Mol Glycidylmethacrylat zur Reaktion bringt. Schließlich besteht eine Variante zur Herstellung darin, daß man das 2,5-Dimethylol-tetrahydrofuran zunächst mit 1 bis 10, insbesondere 2 bis 4 Mol Ethylenoxid und/oder Propylenoxid alkyliert und dann mit Maleinsäureanhydrid verestert und zuletzt mit Glycidylmethacrylat zur Reaktion bringt. Zur Beschleunigung der Additionsreaktion von Ethylenoxid oder Propylenoxid können geeignete Katalysatoren, wie quartäre Ammoniumverbindungen, zugesetzt werden.

Als Ausgangsmaterial zur Herstellung der erfindungsgemäßen Verbindungen verwendet man zweckmäßig handelsübliches 2,5-Dimethylol-tetrahydrofuran. Die Reaktionen können lösungsmittelfrei bei 80 bis 120 °C, eventuell unter Schutzgasatmosphäre, beziehungsweise in einem inerten Lösungsmittel, zum Beispiel Chlorkohlenwasserstoff durchgeführt werden. Bei den so erhältlichen Verbindungen handelt es sich um hochviskose Substanzen. Technisch gut verwendbare Verbindungen können noch eine geringe Säurezahl bis zu etwa 12 aufweisen.

Werden die erfindungsgemäßen Verbindungen als wesentlicher Bestandteil von bei Sauerstoffausschluß erhärtenden Klebstoffen beziehungsweise Dichtungsmitteln eingesetzt, so können diese Mischungen noch weitere Methacrylsäureester enthalten. Hier kommen zum Beispiel in Frage : Ethylenglykoldimethacrylat, Triethylenglykoldimethacrylat, Polyethylenglykoldimethacrylat, Hydroxyethylmethacrylat, Hydroxypropylmethacrylat oder Ethylhexylmethacrylat, Cyclohexylmethacrylat, 5,6-Dihydrodicyclopentadienylmethacrylat, Tetrahydrofurfurylmethacrylat, Dimethacrylsäureester von Dimethyloltricyclodecan.

Ein weiterer wesentlicher Bestandteil der anaerob härtenden Systeme sind die Peroxidinitiatoren. Es handelt sich hier in erster Linie um Hydroperoxide, die sich von Kohlenwasserstoffen ableiten, welche eine Kettenlänge von 3 bis 18 Kohlenstoffatomen aufweisen. Beispielsweise sind geeignet Cumolhydroperoxid, tert.-Butylhydroperoxid, Methylethylketonhydroperoxid, Diisopropylbenzolhydroperoxid. Weiter sind auch solche Perverbindungen geeignet, die bei einer Temperatur zwischen etwa 80 und 140 °C eine Halbwertzeit von 10 Stunden haben.

Hier kommen in Betracht tert.-Butylperbenzoat, Di-tert.-butyl-diperoxyphthalat, 2,5-Dimethyl-2,5-bis-(tert.-butylperoxy)-hexan, Bis-(1-hydroxy-cyclohexyl)-peroxid, tert.-Butylperoxyacetat, 2,5-Dimethylhexyl-2,5-di-(peroxybenzoat), 2,2-Bis-(tert.-butylperoxy)-butan und Di-tert.-butylperoxid.

Die Peroxide bzw. Perverbindungen sollen in einer Menge von 0,1 bis 20 %, insbesondere 1,0 bis 10 %, bezogen auf das Gesamtgemisch, vorhanden sein. Sie werden meist als phlegmatisierte Lösungen oder Pasten eingesetzt, das heißt mit einem relativ geringen Gehalt an inerten Substanzen wie Dimethylphthalat oder Cumol.

Zusätzlich kann ein organisches Amin als Beschleuniger verwendet werden wie etwa substituierte Hydrazide, wie p-Toluolsulfonsäurehydrazid, oder Benzoesäuresulfinimid oder Amine wie N,N-Dimethyl-p-toluidin und Tri-n-butylamin. Amine sollen nur in sehr kleinen Mengen von 0,1 oder bis zu 2,5 Gewichtsprozent verwendet werden.

Zweckmäßig werden diesen Klebstoffen und Dichtungsmitteln Stabilisatoren zugesetzt. Als Stabilisatoren eignen sich Chinon oder Hydrochinon in Konzentrationen von 100 bis 1 000 ppm, vorzugsweise 200 bis 500 ppm, bezogen auf polymerisierbare Anteile. Beschleuniger und Stabilisator müssen in aufeinander abgestimmten Verhältnissen zugesetzt werden, um optimale Eigenschaften der Klebstoffe oder Dichtungsmittel zu erzielen. Im allgemeinen werden sie in Mengen von 0,1 bis 3 Gewichtsprozent, bezogen auf polymerisierbare Anteile, eingesetzt.

Für bestimmte Anwendungszwecke können diese Klebstoffe oder Dichtungsmassen Zusätze an Weichmachern, Verdickungsmitteln oder Farbstoffen enthalten. Die Klebstoffe oder Dichtungsmassen werden durch einen Mischprozeß aller Komponenten bei Raumtemperatur hergestellt und sind in den meisten Fällen Jahre stabil, sofern sie in luftdurchlässigen Gefäßen, wie Polyethylenflaschen, gelagert werden. Bringt man die Klebstoffe oder Dichtungsmittel zwischen Metalloberflächen, so polymerisieren sie rasch unter Bildung einer festen Verbindung der Flächen. Die Vorteile der erfindungsgemäßen Klebstoffe oder Dichtungsmittel sind unter anderem darin zu sehen, daß die zu verbindenden Teile bei Raumtemperatur verklebbar und schon nach kurzer Zeit belastbar sind. Ferner können neben eisenhaltigen Materialien auch Aluminium beziehungsweise Aluminiumlegierungen mit ausreichenden Festigkeiten verklebt werden. Die Wärmefestigkeit der Klebeverbindungen sind hervorragend.

Der erfindungsgemäße Klebstoff ist zum Verbinden von Metallen besonders dann geeignet, wenn hohe Festigkeit bei guter Wärmestabilität der Klebefuge gefordert wird. Die Klebstoffe finden daher in der Technik Anwendung zum Verkleben von Blechen beziehungsweise Metallteilen aus verschiedenen Materialien, zur Befestigung von Lagerwellen, zum Abdichten von Rohrverbindungen und dergleichen mehr. Auffällig ist der relativ schwache Abfall der Festigkeit bei 100 bis 150 °C. Selbst bei 180 °C sind noch beachtliche Drehkraftmomente zu messen.

Beispiel 1a

Bis-methacrylsäureester des 2,5-Dimethylol-tetrahydrofurans. In 400 ml trockenem Tetrahydrofuran wurden 132 g (1 Mol) 2,5-Dimethylol-tetrahydrofuran gelöst und mit 202 g (2 Mol) Triethylamin versetzt. Unter Rühren und Kühlen wurden bei 5 °C innerhalb von 2 Stunden 208 g (2 Mol) Methacrylsäurechlorid zugetropft. Anschließend ließ man die Temperatur für 1 Stunde bei 20 °C und 1 Stunde bei 50 °C. Das Triethylaminhydrochlorid wurde abgesaugt und das Lösungsmittel im Vakuum entfernt. Der Rückstand wurde in Essigester aufgenommen und mit Wasser und Natriumbicarbonatlösung gewaschen. Nach dem Trocknen über Natriumsulfat wurde eingedampft. Es hinterblieb ein zähviskoses farbloses Öl.

Ausbeute : 204 g = 76 %

Analyse : $C_{14}H_{20}O_5$(MG 268,31)

ber. C 62,67 % H 7,51 %

gef. C 62,3 % H 7,32 %

Der Bis-methacrylsäureester des 2,5-Dimethylol-tetrahydrofurans wurde auch durch direkte Veresterung mit Methacrylsäure in Tetrachlorkohlenstoff als Lösungsmittel und durch Umesterung mit Methacrylsäuremethylester im Überschuß hergestellt.

Beispiel 2a

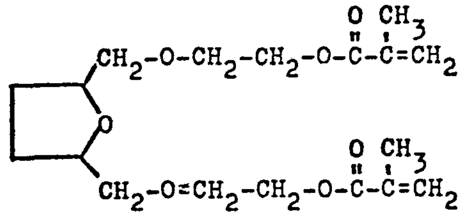

110 g (0,5 Mol) ethoxyliertes 2,5-Dimethylol-tetrahydrofuran (OH-Zahl 409,25) wurden in 300 ml Tetrahydrofuran gelöst und mit 101 g (1 Mol) Triethylamin versetzt. Unter Rühren und Kühlen wurden bei 5 °C 104 g (1 Mol) Methacrylsäurechlorid zugetropft. Die Nachreaktion und Aufarbeitung erfolgte wie im vorstehenden Beispiel beschrieben.

Der erhaltene Bis-methacrylsäureester ist ein zähviskoses farbloses Öl.

Ausbeute : 152 g = 80 % d. Th.

Analyse : $C_{18}H_{28}O_7$ (MG 356,42)

ber. C 60,66 % H 7,92 %

gef. C 59,3 % H 7,94 %

Alternativ wurde der Bis-methylacrylsäureester durch direkte Veresterung mit Methacrylsäure in Tetrachlorkohlenstoff hergestellt.

Beispiel 3a

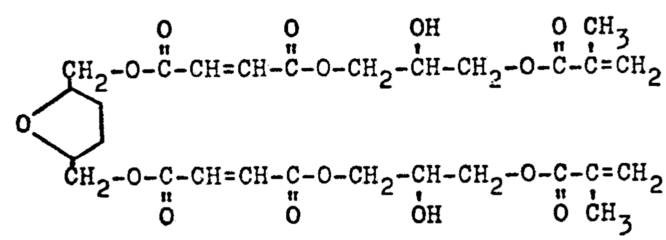

132 g (1 Mol) 2,5-Dimethylol-tetrahydrofuran und 196 g (2 Mol) Maleinsäureanhydrid wurden auf 100 °C erhitzt. Nach dreistündiger Reaktionszeit lag die Säurezahl bei 352 (ber. 342). Dieser Bis-halbester wurde anschließend bei 80 °C mit 284 g (2 Mol) Glycidylmethacrylat umgesetzt. Katalysiert wurde die Reaktion durch 0,6 g (0,1 %) Benzyltrimethylammonium-Methoxid. Der entstehende Bis-methacrylsäureester wurde durch Zugabe von 600 mg Hydrochinon stabilisiert. Nach sechsstündiger Reaktionszeit lag die Säurezahl bei 12, die Reaktion wurde dann abgebrochen. Es wurde ein zähviskoses farbloses Öl erhalten.

Ausbeute : 612 g (100 % d. Th.)

Analyse : $C_{28}H_{36}O_{15}$ (MG 612,60)

ber. C 54,90 % H 5,92 %

gef. C 54,6 % H 5,80 %

Beispiel 4a

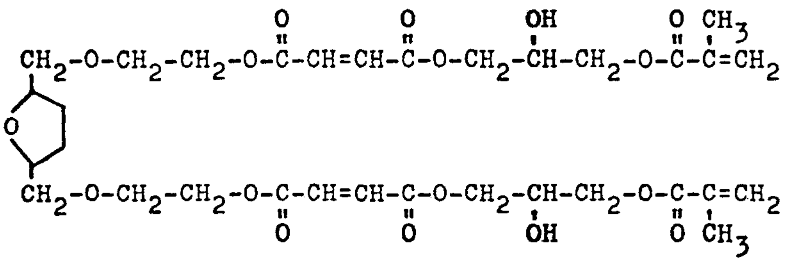

165 g (0,75 Mol) ethoxyliertes 2,5-Dimethylol-tetrahydrofuran (OH-Zahl 409,25) und 147 g (1,5 Mol) Maleinsäureanhydrid wurden auf 100 °C erhitzt. Nach vierstündiger Reaktionszeit lag die Säurezahl bei 287 (ber. 269). Der Bis-halbester wurde anschließend in Gegenwart von 0,5 g Benzyltrimethylammonium-methoxid und 0,5 g Hydrochinon mit 213 g (1,5 Mol) Glycidylmethacrylat bei 80 °C umgesetzt. Nach sechsstündiger Reaktionszeit lag die Säurezahl bei 14. Die Reaktion wurde abgebrochen. Es resultierte ein zähviskoses farbloses Öl.

Ausbeute : 525 g (100 % d. Th.)

Analyse : $C_{32}H_{44}O_{17}$ (MG 700,69)

ber. C 54,85 % H 6,33 %

gef. C 53,8 % H 5,83 %

Beispiele 1b bis 4b

Klebemischungen mit den Bis-methacrylaten. Die Bis-methylacrylsäureester der Beispiele 1a bis 4a wurden mit Hydroxyethylmethacrylat im Verhältnis 1 : 1 vermischt. Diese Monomerengemische wurden sodann mit 200 ppm Hydrochinon, 1 % p-Toluolsulfonsäurehydrazid, 0,5 % N,N-Dimethyl-p-toluidin und 3 % Cumolhydroperoxid (70 %ig in Cumol) versetzt. Die resultierenden anaerob härtenden Klebstoffe wurden hinsichtlich ihrer Eigenschaften überprüft. Die Stabilität aller Mischungen lag oberhalb 30 Min.

Bei der Stabilitätsprüfung wurde ein 10 cm langes und 10 mm weites Reagenzglas zu 9/10 mit der Mischung nach Beispiel 1b bis 4b gefüllt und in ein auf 80 °C gehaltenes Bad eingehängt. Die Zeitspanne vom Einhängen bis zur ersten Gelbildung wurde gemessen. Alle Proben waren länger als 30 Minuten gelfrei. Diese Werte bedeuten, daß die Produkte bei Raumtemperatur in der Regel mehr als ein Jahr unverändert haltbar sind.

Vergleichsversuch

Der Bis-methacrylsäureester von propoxyliertem Diphenylolpropan (DE-AS 22 02 040) wurde mit Hydroxyethylmethacrylat im Verhältnis 1 : 1 gemischt. Weiter wurden die gleichen Zusätze gemacht wie in den vorstehenden Beispielen 1b bis 4b.

In der nachfolgenden Tabelle ist in Abhängigkeit von Beispiel 1b bis 4b sowie dem Vergleichsversuch angegeben das an 5 Proben ermittelte durchschnittliche Drehmoment an M 10 × 30 Eisenschrauben und M 10 Muttern sowie die durchschnittliche Druckscherfestigkeit nach DIN-Entwurf Nr. 54452. Die Angaben unter 150 °C (Drehmoment) und 180 °C (Druckscherfestigkeit) bedeuten, daß die Proben nach 3tägiger Lagerung bei Raumtemperatur für 3 Stunden auf 150 °C beziehungsweise 180 °C erwärmt wurden und dann die Messung bei dieser Temperatur vorgenommen wurde.

Tabelle

Eigenschaften der hergestellten Klebeverbindung

| Mischung gemäß Beispiel Nr. | Drehmomente in Nm | | | Druckscherfestigkeiten N/mm² | |
|---|---|---|---|---|---|
| | 20° C nach 3 Stunden | 20° C nach 24 Stunden | 150° C nach 3 Tagen | 20° C nach 3 Tagen | 180° C nach 3 Tagen |
| 1b | 40 | 65 | 24 | 35 | 18 |
| 2b | 30 | 55 | 20 | 30 | 15 |
| 3b | 25 | 68 | 30 | 45 | 20 |
| 4b | 38 | 70 | 32 | 35 | 15 |
| Vergleichs-versuch | 40 | 66 | 14 | 35 | 10 |

**Ansprüche**

1. Bis-methacrylsäureester der allgemeinen Formel

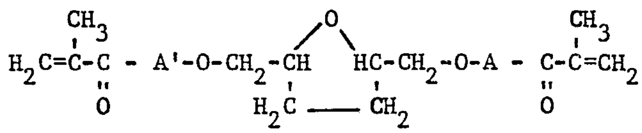

in der A beziehungsweise A′ folgende Bedeutung haben

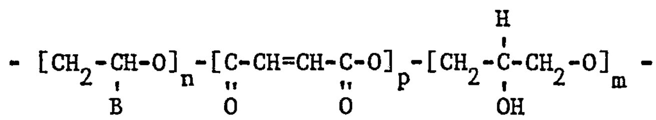

wobei

B = $CH_3$ oder H ist und n, p, m ganze Zahlen sind, die die Werte n = 0 bis 5 und m = 0 oder 1 und p = 0 oder 1 annehmen können.

2. Verfahren zur Herstellung der Verbindung gemäß Anspruch 1, bei denen n, p und m 0 bedeuten, dadurch gekennzeichnet, daß man in an sich bekannter Weise 2,5-Dimethylol-tetrahydrofuran mit Methacrylsäure beziehungsweise deren Chlorid beziehungsweise deren Methylester umsetzt.

3. Verfahren zur Herstellung der Verbindungen gemäß Anspruch 1, bei denen n 1 und p und m 0 bedeuten, dadurch gekennzeichnet, daß man in an sich bekannter Weise

a) 2,5-Dimethylol-tetrahydrofuran mit Ethylenoxid und/oder Propylenoxid und dann

b) mit Methacrylsäure beziehungsweise deren chlorid beziehungsweise deren Methylester umsetzt.

4. Verfahren zur Herstellung eines Bis-methacrylsäureesters gemäß Anspruch 1, dadurch gekennzeichnet, daß man in an sich bekannter Weise 1 Mol 2,5-Dimethylol-tetrahydrofuran mit 1 bis 2 Mol Maleinsäureanhydrid umsetzt und dann mit 2 Mol Glycidylmethacrylat zur Reaktion bringt.

5. Verfahren zur Herstellung der Verbindungen gemäß Anspruch 1, bei denen n 1 bis 5 und p und n 1 bedeuten, dadurch gekennzeichnet, daß man das 2,5-Dimethyloltetrahydrofuran in an sich bekannter Weise zunächst mit 1 bis 10 Mol Ethylenoxid und/oder Propylenoxid dann mit Maleinsäureanhydrid und schließlich mit Glycidylmethacrylat umsetzt.

6. Bei Sauerstoffausschluß erhärtende Klebstoffe beziehungsweise Dichtungsmittel enthaltend als wesentlichen Bestandteil Verbindungen gemäß Anspruch 1.

7. Bei Sauerstoffausschluß erhärtende Klebstoffe beziehungsweise Bindemittel enthaltend als wesentlichen Bestandteil ein und/oder mehrere Verfahrensprodukte nach einem der Ansprüche 2 bis 5.

**Claims**

1. Bis-methacrylic acid esters corresponding to the following general formula

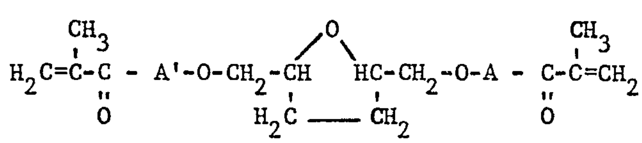

in which A and A′ have the following meaning

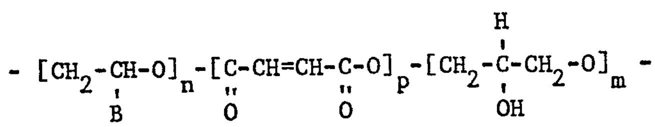

where

B = $CH_3$ or H and n, p and m are integers which may respectively assume values of from 0 to 5, 0 or 1 and 0 or 1.

2. A process for producing the compounds claimed in Claim 1, in which n, p and m = 0, characterized in that 2,5-dimethylol tetrahydrofuran is reacted in known manner with methacrylic acid, its chloride or its methyl ester.

3. A process for producing the compounds claimed in Claim 1, in which n = 1 and p and m = 0, characterized in that, using methods known *per se,*

a) 2,5-dimethylol tetrahydrofuran is reacted with ethylene oxide and/or propylene oxide and then

b) with methacrylic acid, its chloride or its methyl ester.

4. A process for producing a bis-methacrylic acid ester of the type claimed in Claim 1, characterized in that, using methods known *per se,* 1 mole of 2,5-dimethylol tetrahydrofuran is reacted with 1 to 2 moles of maleic acid anhydride and then with 2 moles of glycidyl methacrylate.

5. A process for producing the compounds claimed in Claim 1, in which n = 1 to 5 and p and n = 1, characterized in that, using methods known *per se,* 2,5-dimethylol tetrahydrofuran is reacted in known manner first with 1 to 10 moles of ethylene oxide and/or propylene oxide, then with maleic acid anhydride and finally with glycidyl methacrylate.

6. Adhesives and sealing compounds hardening in the absence of oxygen and containing as their principal constituent compounds of the type claimed in Claim 1.

7. Adhesives and binders hardening in the absence of oxygen and containing as their principal constituent one and/or more of the compounds obtained by the process claimed in any of Claims 2 to 5.

**Revendications**

1. Bis-méthacrylates de formule générale

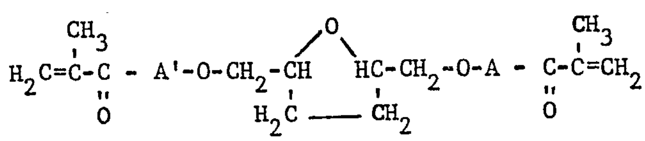

dans laquelle A et A' ont la signification suivante

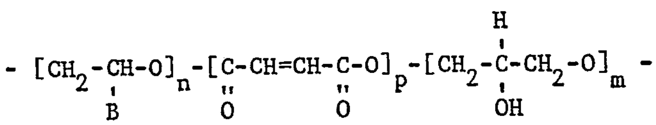

où

B = $CH_3$ ou H et n, p, m sont des nombres entiers et peuvent prendre les valeurs suivantes n = 0 à 5 et m = 0 ou 1 et p = 0 ou 1.

2. Procédé pour la fabrication des composés selon la revendication 1, dans lesquels n, p et m sont égaux à 0, caractérisé en ce que l'on fait réagir de manière connue le diméthylol-2,5-tétrahydrofuranne avec l'acide méthacrylique ou son chlorure ou son ester de méthyle.

3. Procédé pour la fabrication des composés selon la revendication 1, dans lesquels n est égal à 1 et p et m sont égaux à 0, caractérisé en ce que l'on fait réagir de manière connue

a) le diméthylol-2,5-tétrahydrofuranne avec l'oxyde d'éthylène et/ou l'oxyde de propylène et ensuite

b) avec l'acide méthacrylique ou son chlorure ou son ester de méthyle.

4. Procédé pour la fabrication d'un bis-méthacrylate selon la revendication 1, caractérisé en ce que l'on fait réagir de manière connue 1 mol de diméthylol-2,5-tétrahydrofuranne avec 1 à 2 mol d'anhydride maléique et ensuite on fait réagir avec 2 mol de méthacrylate de glycidyle.

5. Procédé pour la fabrication des composés selon la revendication 1, dans lesquels n est compris entre 1 et 5 et p et n sont égaux à 1, caractérisé en ce que l'on fait réagir de manière connue le diméthylol-2,5-tétrahydrofuranne d'abord avec 1 à 10 mol d'oxyde d'éthylène et/ou d'oxyde de propylène, ensuite avec l'anhydride maléique et enfin avec le méthacrylate de glycidyle.

6. Colles ou matières d'obturation durcissant à l'abri de l'oxygène, contenant comme constituants essentiels des composés selon la revendication 1.

7. Colles ou adhésifs durcissant à l'abri de l'oxygène, contenant comme constituants essentiels un ou plusieurs produits du procédé selon l'une des revendications 2 à 5.